# EUROPÄISCHE PATENTSCHRIFT

(11) **EP 0 692 269 B1**
(45) Veröffentlichungstag und Bekanntmachung des Hinweises auf die Patenterteilung: **22.03.2000**
(21) Anmeldenummer: 95110613.7
(22) Anmeldetag: 07.07.1995
(51) Int. Cl.: A61M 1/34

(54) **Hämo(dia)filtrationsvorrichtung mit Filtratflusssteuerung**
Haemo(dia)filtration device with filtrate flow control
Dispositif d'hémo(dia)filtration avec contrôle du débit de filtrat

(30) Priorität: 13.07.1994 DE 4424693
(43) Veröffentlichungstag der Anmeldung: 17.01.1996
(73) Patentinhaber: Fresenius Medical Care Deutschland GmbH, 61350 Bad Homburg v.d.H. (DE)
(72) Erfinder: Nederlof, Bernd, D-66606 St. Wendel (DE)
(74) Vertreter: Luderschmidt, Schüler & Partner GbR

(56) Entgegenhaltungen:
- EP-A- 0 270 794
- DE-A- 3 444 671
- FR-A- 2 404 439
- US-A- 4 024 059

## Beschreibung

Die Erfindung betrifft eine Hämo(dia)filtrationsvorrichtung mit einem Blutfilter, der durch eine Membran in zwei Kammern geteilt ist, wobei die erste Kammer in einen Dialysierflüssigkeitsweg und die zweite Kammer in einen Blutweg geschaltet ist, der Dialysierflüssigkeitsweg eine Zuleitung, die sich von einer Einrichtung zur Bereitstellung von Dialysierflüssigkeit bis zum Blutfilter erstreckt und in die eine erste Bilanziereinheit eingeschaltet ist, und eine Ableitung aufweist, die sich vom Blutfilter zu einem Abfluß erstreckt und in die eine zweite Bilanziereinheit eingeschaltet ist, mit einer Pumpe zum Fördern der Dialysierflüssigkeit im geschlossenen Dialysierflüssigkeitssystem, eine die Zuleitung mit der Ableitung des Dialysierflüssigkeitsweges verbindenden Bypass-Leitung, in die ein Bypass-Ventil eingeschaltet ist, einer Ultrafiltrationseinrichtung, einem in der Zuleitung zwischen der ersten Bilanziereinheit und dem Blutfilter angeordnetem ersten Sterilfilter, der durch eine Keime zurückhaltende Membran in eine erste Kammer und eine zweite Kammer geteilt ist, und einem zweiten Sterilfilter, der durch eine Keime zurückhaltende Membran in eine erste Kammer und eine zweite Kammer geteilt ist.

Bei der Hämofiltration wird Blut an der Membran eines Hämofilters vorbeigeleitet, wobei ein Teil des Serums durch die Membran abgezogen und durch eine sterile Substitutionsflüssigkeit ersetzt wird, die entweder stromauf des Blutfilters (Prädilution) oder stromab des Blutfilters (Postdilution) dem extrakorporalen Blutweg zugesetzt wird. Zusätzlich wird bei der Hämodiafiltration noch die übliche Hämodialyse durchgeführt, d.h., es wird an der Membran des Hämodialysators Dialysierflüssigkeit vorbeigeleitet, so daß über die Membran hinweg ein Austausch von harnpflichtigen Sustanzen erfolgen kann.

Die Dialysierflüssigkeit kann on-line aus Frischwasser und einem Elektrolytkonzentrat und die Substitutionsflüssigkeit on-line aus der Dialysierflüssigkeit hergestellt werden. Das Elektrolytkonzentrat ist zwar in der Regel eigensteril und das Frischwasser weist üblicherweise keine Keime auf, es ist jedoch nicht sichergestellt, daß die on-line hergestellte Dialysierflüssigkeit absolut steril und pyrogenfrei ist, weshalb die Dialysierflüssigkeit zur Herstellung der Substitutionsflüssigkeit in den sterilen und pyrogenfreien Zustand überführt wird. Dazu wird stromauf des Blutfilters mit einer Leitung Dialysierflüssigkeit entnommen, wobei in diese Leitung mindestens ein Sterilfilter eingeschaltet ist.

Eine derartige Vorrichtung ist beispielsweise aus der DE 34 44 671 A bekannt.

Diese genannte Vorrichtung hat jedoch durch die sogenannte "dead end"-Anordnung des Sterilfilters in der Substituatleitung den Nachteil, daß sich im Laufe der Zeit Partikel und andere Substanzen, z.B. eingeschleppte Keime und Pyrogene vor der Membran des Sterilfilters ansammeln. Dies ist insbesondere dann gefährlich, wenn durch eine Ruptur diese Substanzen schlagartig in den Sterilbereich eingeschleppt werden und die Substitutionsflüssigkeit damit kontaminiert wird.

Weiterhin bestehen durch die Substituatleitung mit den darin angeordneten zusätzlichen Einrichtungen, wie beispielsweise Substituatpumpe und Belüftungseinrichtung, weitere Kontaminationsrisiken, und die Gestaltung der Substituatleitung selbst ist, nicht zuletzt durch diese zusätzlichen Einrichtungen aufwendig und damit kostenintensiv.

Es ist ferner aus dem Aufsatz von v. Albertini et al., "Serial and parallel pairs of dialyzers for high-efficiency treatment", in Kidney International 31(1), 1987, Seite 247, bekannt, zwei Blutfilter zur Erhöhung der Reinigungswirkung in Reihe oder parallel anzuordnen. Eine Vorrichtung zur exakten Steuerung und Überwachung des Filtrat- und Substituatflusses ist dieser Veröffentlichung nicht zu entnehmen.

Für eine wirkungsvolle und sichere Betätigung dieser Vorrichtungen sind jedoch aufwendige und komplizierte Vorrichtungen zur Steuerung und Überwachung des Verhältnisses von Substituat und Filtrat notwendig. Auch ist die Messung von Undichtigkeit der Membran des Blutfilters nur mit großem Aufwand und äußerst umständlich möglich.

Der Erfindung liegt daher die Aufgabe zugrunde, ausgehend von einer Hämo(dia)filtrationsvorrichtung der eingangs genannten Art eine einfachere, effektivere und sicherere Hämo(dia)filtrationsvorrichtung zur Verfügung zu stellen, bei der ein Zusetzen der Sterilfilter mit Keimen oder Pyrogenen weitgehend verhindert wird, das Verhältnis von Filtrat und Substituat einfach und sicher steuerbar und überwachbar ist und eine einfache und sichere Messung auf Undichtigkeit der Membran des Blutfilters möglich ist.

Die Lösung der Aufgabe erfolgt dadurch, daß der zweite Sterilfilter ein weiterer Blutfilter ist, welcher in der Zuleitung zwischen erstem Sterilfilter und erstem Blutfilter angeordnet ist, daß die erste Kammer des zweiten Blutfilters in den Dialysierflüssigkeitsweg und die zweite Kammer in den Blutweg geschaltet ist, daß die erste Kammer des zweiten Blutfilters über eine erste Verbindungsleitung mit der ersten Kammer des ersten Blutfilters verbunden ist und die zweite Kammer des zweiten Blutfilters über eine zweite Verbindungsleitung mit der zweiten Kammer des ersten Blutfilters verbunden ist, und daß in einer der Verbindungsleitungen zwischen den Blutfiltern mindestens eine Pumpe zur Erzeugung einer Druckdifferenz zwischen den Blutfiltern derart angeordnet ist, daß in dem einen Blutfilter ein positiver Transmembrandruck in Richtung des Blutweges und in dem anderen Blutfilter ein negativer Transmembrandruck in Richtung des Blutweges erzeugt wird.

Durch diese erfindungsgemäße Anordnung eines zweiten Blutfilters wird zum einen eine effektivere Hämodialyse ermöglicht, zum anderen kann auch auf eine aufwendige Substituatleitung mit darin angeordneten zusätzlichen Einrichtungen, welche üblicherweise das Substituat dem Blutweg zuführt, verzichtet werden, da die Dialysierflüssigkeit als Substituatlösung durch die Membran des einen Blutfilters direkt in das Blut überführt wird. Damit ist eine Kontamination des Substituates beim Durchgang durch die Substituatleitung nicht mehr möglich. Des weiteren lassen sich durch geeignete Vorrichtungen, welche beispielsweise die Druckdifferenz zwischen den Blutfiltern bestimmen und steuern, Filtrat- und Substituatfluß genau bestimmen und individuell einstellen. Auch ist eine einfache und effektive Überwachung der Membranen der beiden Blutfilter durch eine intermittierende Schaltung der Druckdifferenz zwischen den Blutfiltern mittels eines Blutleckdetektors möglich.

Eine besonders vorteilhafte Weiterbildung der Erfindung sieht vor, den ersten Sterilfilter in die Zuleitung einzuschalten und die erste Kammer des ersten Sterilfilters zumindest zeitweise in Durchfluß zu schalten. Vorteilhafterweise wird dazu die erste Kammer des ersten Sterilfilters mit der zur Ableitung führenden Bypassleitung verbunden.

Durch diese Anordnung des ersten Sterilfilters erfolgt eine sterilisierend wirkende Filtration der Dialysierflüssigkeit, so daß den nachgeschalteten Blutfiltern eine absolut sterile Dialysierflüssigkeit zugeführt wird. Durch Öffnen des Bypassventils, welches sowohl während der Behandlung als auch während des Spülbetriebes der gesamten Anordnung geöffnet werden kann, fließt die Dialysierflüssigkeit aus der ersten Kammer des Sterilfilters ab und reißt dort die an der Membran befindlichen Pyrogene und Teilchen in die Ableitung und von dort in den Abfluß mit. Das Freispülen der Membran kann dabei in vorbestimmten Abständen erfolgen, so daß das Ansammeln von Keimen und Pyrogenen an der Membran zuverlässig verhindert wird. Da das Zusetzen der Membran mit der daraus resultierenden Erhöhung des Transmembrandruckes ein Hauptgrund für das Entstehen einer Ruptur ist, wird das Entstehen einer Ruptur somit unwahrscheinlicher, so daß die Sterilität der Dialysierflüssigkeit gewährleistet ist und die Sicherheit der Hämo(dia)filtrationsvorrichtung verbessert wird.

Erfindungsgemäß weist die Vorrichtung zur Erzeugung einer Druckdifferenz zwischen den Blutfiltern mindestens eine Pumpe auf. Damit ist zum einen eine gezielte Steuerung des Substituatflusses und des Filtratflusses durch die Membranen der Blutfilter hindurch möglich, und zum anderen ist eine Umkehr des Filtrat- und Substituatflusses möglich. Durch eine intermittierende Schaltung des Filtrat- und Substituatflusses läßt sich zudem die Dichtheit der Membranen der Blutfilter, beispielsweise mittels eines Blutleckdetektors äußerst einfach und wirkungsvoll überwachen. Diese Pumpe kann entweder in die Verbindungsleitung zwischen den ersten Kammern der Blutfilter oder in die Verbindungsleitung zwischen den zweiten Kammern der Blutfilter, also entweder im Dialysierflüssigkeitsweg oder im Blutweg eingeschaltet werden. Wird diese Pumpe jedoch in den Dialysierflüssigkeitsweg eingeschaltet, so muß diese eine nicht okkludierende Pumpe sein.

Eine vorteilhafte Weiterbildung sieht vor, eine zweite Bypassleitung von der Verbindungsleitung zwischen den ersten Kammern der Blutfilter zur Ableitung des Dialysierflüssigkeitsweges zu führen. Eine erste Pumpe wird dann stromab des Anschlusses dieser Bypassleitung an die Verbindungsleitung und stromauf des ersten Blutfilters in die erste Verbindungsleitung zwischen den ersten Kammern der Blutfilter eingeschaltet, und eine zweite Pumpe wird stromab des ersten Blutfilters und stromauf des Anschlusses der zweiten Bypassleitung in die Ableitung eingeschaltet. Damit ist ein ungestörter Fluß der Dialysierflüssigkeit durch den Dialysierflüssigkeitsweg und den zweiten Blutfilter möglich. Bei Bedarf kann dann der erste Blutfilter in den Dialysierflüssigkeitsweg eingeschaltet werden und durch unterschiedliche Pumpraten der beiden Pumpen eine Druckdifferenz zwischen erstem Blutfilter und zweiten Blutfilter erzeugt werden.

Insgesamt wird somit eine effektive und einfache Hämo(dia)filtrationsvorrichtung bereitgestellt, die zum einen eine doppelte Sicherheit gegen Kontamination des Blutes bietet, da die Dialysierflüssigkeit als Substituatlösung zweifach gefiltert in das Blut überführt wird, und bei der zum anderen eine Kontamination der Substituatlösung nicht möglich ist, da diese direkt in das Blut überführt wird und nicht mehr durch eine Substituatleitung mit darin angeordneten zusätzlichen Einrichtungen dem Blutweg zugeführt wird. Weiterhin wird durch das zumindest teilweise Freispülen der Membran des ersten Sterilfilters und durch das ständige Freispülen der Membranen der beiden Blutfilter eine Anlagerung von Keimen oder Pyrogenen an diesen Membranen verhindert, so daß die Gefahr einer Ruptur an einer dieser Membranen erheblich reduziert ist. Des weiteren ist die Intaktheit dieser Membranen einfach und wirkungsvoll zu prüfen, so daß eine Hämo(dia)filtrationsvorrichtung mit einem hohen Sicherheitsniveau bereitgestellt wird, und das Verhältnis von Substituat und Filtrat ist individuell einstellbar.

Weitere Einzelheiten, Merkmale und Vorteile der Erfindung sind anhand der nachfolgenden Beschreibung von drei Ausführungsbeispielen unter Bezugnahme auf die Zeichnungen näher erläutert, in denen
- Figur 1: ein erstes Ausführungsbeispiel der Filtratflußsteuerung in einer schematischen Darstellung zeigt,
- Figur 2: eine schematische Darstellung eines zweiten Ausführungsbeispieles zeigt, und
- Figur 3: eine schematische Darstellung eines dritten Ausführungsbeispieles zeigt.

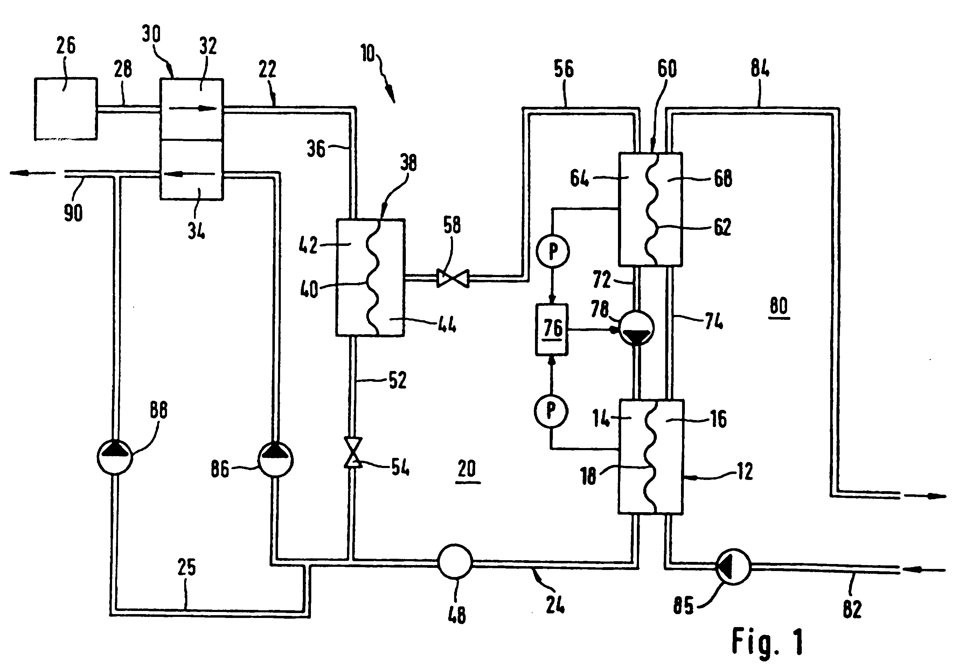

In Figur 1 ist mit 10 eine Diafiltrationsvorrichtung gezeigt, die einen ersten Blutfilter 12, der durch eine Membran 18 in eine von Dialysierflüssigkeit durchflossene erste Kammer 14 und eine von Blut durchflossene zweite Kammer 16 getrennt ist, und einen zweiten Blutfilter 60 aufweist, der durch eine Membran 62 in eine von Dialysierflüssigkeit durchflossene erste Kammer 64 und eine von Blut durchflossene zweite Kammer 68 getrennt ist.

Die erste Kammer 14 des ersten Blutfilters 12 und die erste Kammer 64 des zweiten Blutfilters 60 sind in einen Dialysierflüssigkeitsweg 20 eingeschaltet, der aus einer Zuleitung 22 und einer Ableitung 24 besteht, und die zweite Kammer 16 des ersten Blutfilters 12 und die zweite Kammer 68 des zweiten Blutfilters 60 sind in einen Blutweg 80 eingeschaltet.

Die Zuleitung 22 besteht aus einem ersten Zuleitungsabschnitt 28, einem zweiten Zuleitungsabschnitt 36 und einem dritten Zuleitungsabschnitt 56 und verbindet eine Dialysierflüssigkeitsquelle 26 mit der ersten Kammer 64 des zweiten Blutfilters 60.

Der erste Zuleitungsabschnitt 28 verbindet die Dialysierflüssigkeitsquelle 26 mit der ersten Kammer 32 einer Bilanzierungsvorrichtung 30. Die erste Kammer 32 der Bilanzierungsvorrichtung 30 ist über den zweiten Zuleitungsabschnitt 36 mit der ersten Kammer 42 eines Sterilfilters 38 verbunden. Der Sterilfilter 38 ist durch eine Membran 40 in eine erste Kammer 42 und eine zweite Kammer 44 unterteilt. Der Ausgang der ersten Kammer 42 des Sterilfilters 38 ist mit einer Bypass-Leitung 52 verbunden, in die ein Bypass-Ventil 54 eingeschaltet ist und die mit der Ableitung 24 verbunden ist.

Von der zweiten Kammer 44 des Sterilfilters 38 geht der dritte Zuleitungsabschnitt 56 ab und ist mit der ersten Kammer 64 des zweiten Blutfilters 60 verbunden. In dem dritten Zuleitungsabschnitt 56 ist ein Ventil 58 eingeschaltet. Die erste Kammer 64 des zweiten Blutfilters 60 ist über die erste Verbindungsleitung 72 mit der ersten Kammer 14 des ersten Blutfilters 12 verbunden und die zweite Kammer 68 des zweiten Blutfilters 60 ist über die zweite Verbindungsleitung 74 mit der zweiten Kammer 16 des ersten Blutfilters 12 verbunden.

In die erste Verbindungsleitung 72 zwischen erster Kammer 64 des zweiten Blutfilters 60 und der ersten Kammer 14 des ersten Blutfilters 12 ist eine nicht okkludierende Pumpe 78 eingeschaltet.

Vom Ausgang der ersten Kammer 14 des ersten Blutfilters 12 führt die Ableitung 24 zur zweiten Bilanziereinheit 34 der Bilanzierungsvorrichtung 30. In die Ableitung 24 ist ein Blutleckdetektor 48 und eine Dialysierflüssigkeitspumpe 86 eingeschaltet, des weiteren geht von der Ableitung 24 eine Ultrafiltratleitung 25 ab, in die eine Ultrafiltrationspumpe 88 eingeschaltet ist. Die Ultrafiltratleitung 25 führt zu einem Abfluß 90, an dem auch der Ausgang der zweiten Bilanziereinheit 34 der Bilanzierungsvorrichtung 30 angeschlossen ist.

Die zweite Kammer 16 des ersten Blutfilters 12 und die zweite Kammer 68 des zweiten Blutfilters 60 sind dergestalt in den Blutweg 80 eingeschaltet, daß eine vom Patienten kommende Blutzuleitung 82 an den Eingang der ersten Kammer 16 angeschlossen ist, der Ausgang der ersten Kammer 16 des ersten Blutfilters 12 durch die zweite Verbindungsleitung 74 an den Eingang der zweiten Kammer 68 des zweiten Blutfilters 60 angeschlossen ist, und eine Blutableitung 84 an den Ausgang der zweiten Kammer 68 des zweiten Blutfilters 60 angeschlossen ist. Diese Blutableitung 84 führt das Blut zurück zum Patienten.

Von der Dialysierflüssigkeitsquelle 26 wird frische Dialysierflüssigkeit durch den ersten Zuleitungsabschnitt 28 der ersten Kammer 32 der Bilanzierungsvorrichtung 30 zugeführt. Diese erste Bilanziereinheit 32 ist an die Zuleitung 22 des Dialysierflüssigkeitsweges 20 angeschlossen, so daß die frische Dialysierflüssigkeit von der ersten Bilanziereinheit 32 durch den zweiten Zuleitungsabschnitt 36, den Sterilfilter 38 und den dritten Zuleitungsabschnitt 56 zur ersten Kammer 64 des zweiten Blutfilters 60 geleitet wird. Von dort wird es durch die erste Verbindungsleitung 72 zur ersten Kammer 14 des ersten Blutfilters 12 geleitet. Beim Durchgang durch die Membran 40 des Sterilfilters 38 erfolgt eine sterilisierend wirkende Filtration der Dialysierflüssigkeit, so daß eine vollständig sterilisierte Dialysierflüssigkeit in die erste Kammer 64 des zweiten Blutfilters 60 eingeleitet wird. Der Ausgang der ersten Kammer 14 des ersten Blutfilters 12 ist an die Ableitung 24 angeschlossen, welche zu der zweiten Bilanziereinheit 34 der Bilanzierungsvorrichtung 30 führt. In diese Ableitung 24 ist eine Dialysierflüssigkeitspumpe 86 angeordnet, welche die Dialysierflüssigkeit in dem Dialysierflüssigkeitsweg 20 von der ersten Bilanziereinheit 32 zur zweiten Bilanziereinheit 34 der Bilanzierungsvorrichtung 30 fördert. Die Bilanzierungsvorrichtung 30 ist dabei so ausgebildet, daß eine durch die zweite Bilanziereinheit 34 in den Abfluß 90 strömende Menge an verbrauchter Dialysierflüssigkeit durch eine gleiche Menge frischer Dialysierflüssigkeit ersetzt wird, die durch die erste Bilanziereinheit 32 in die Zuleitung 22 gefördert wird.

Die durch die frische Dialysierflüssigkeit in die Zuleitung 22 eingeschleppten Keime und Pyrogene werden durch die Membran 40 des Sterilfilters 38 gefiltert und setzen sich an dieser ab. Durch Öffnen des Bypass-Ventils 54 in der Bypass-Leitung 52, die eine Verbindung von der ersten Kammer 42 zur Ableitung 24 herstellt, erfolgt eine Durchspülung der ersten Kammer 42 mit Dialysierflüssigkeit. Dieses bewirkt, daß die an der Membran 40 abgesetzten Partikel, Keime und Pyrogene durch die an dieser vorbeiströmenden Dialysierflüssigkeit mitgerissen werden und über die Bypass-Leitung 52, die Ableitung 24 durch die zweite Bilanziereinheit 34 hindurch in den Abfluß 90 gespült werden. Damit ist ein wirkungsvolles Freispülen der Membran 40 des Sterilfilters 38 möglich, so daß die Gefahr einer Ruptur erheblich verringert und die Sterilität der Dialysierflüssigkeit gewährleistet ist und damit diese Anordnung eine hohe Betriebssicherheit aufweist.

Weiterhin ist eine von der Ableitung 24 abgehende Ultrafiltratleitung 25 vorgesehen, welche ebenfalls zum Abfluß 90 führt und in die eine Ultrafiltratpumpe 88 eingeschaltet ist. Durch Betätigen der Ultrafiltratpumpe 88 kann zusätzlich Flüssigkeit aus dem hydraulisch geschlossenen Dialysierflüssigkeitsweg entfernt werden, welche dann durch die Membran 18 des ersten Blutfilters 12 und/oder durch die Membran 62 des zweiten Blutfilters 60 aus dem durch die zweite Kammer 16 des ersten Blufilters 12 und die zweite Kammer 68 des zweiten Blutfilters 60 strömenden Blut nachgezogen wird.

Die Substituatlösung besteht aus der frischen, durch den Sterilfilter 38 gefilterten Dialysierflüssigkeit und wird bei dem vorliegenden Ausführungsbeispiel über die Membran 62 des zweiten Blutfilters 60 ins Blut überführt. Die Pumpe 78 in der ersten Verbindungsleitung 72 zwischen erster Kammer 64 des zweiten Blutfilters 60 und der ersten Kammer 14 des ersten Blutfilters 12 wird in ihrer Förderrichtung und Förderrate so betrieben, daß in der ersten Kammer 64 des zweiten Blutfilters 60 ein positiver Transmembrandruck in Richtung des Blutweges 80 und in der ersten Kammer 14 des ersten Blutfilters 12 ein negativer Transmembrandruck in Richtung des Blutweges 80 erzeugt wird. Dadurch wird die Dialysierflüssigkeit als Substituat durch die Membran 62 des zweiten Blutfilters 60 hindurch in das Blut gefiltert und Flüssigkeit durch die Membran 18 des ersten Blutfilters 12 hindurch aus dem in der zweiten Kammer 16 strömenden Blut in die erste Kammer 14 gezogen, wobei beide Flüssigkeitsmengen wegen des geschlossenen Kreislaufs innerhalb des Bilanziereinheit systems gleich sind.

Durch eine Vorrichtung 76 zur Überwachung dieser Druckdifferenz läßt sich der Substituatfluß durch die Membran 62 des zweiten Blutfilters 60 und der Filtratfluß durch die Membran 18 des ersten Blutfilters 12 genau bestimmen, und hierdurch an der Pumpe 78 zur Steuerung dieser Druckdifferenz Filtrat- und Substituatfluß individuell einstellen. Die Vorrichtung 76 weist jeweils Drucksensoren an den Blutfiltern 12 und 60 auf, deren Meßwerte kontinuierlich zur Bestimmung des jeweiligen Filtratflusses durch die Membran 18 und 62 herangezogen werden. Der Filtratfluß läßt sich dabei anhand der bekannten Filterkenndaten (Durchlässigkeit, Ultrafiltrationsrate etc.) bei einem bestimmten Druckwert und bekannter Fluidrate bestimmen.

Durch diese Anordnung wird zum einen eine doppelte Sicherheit gegen Kontamination des Blutes erreicht, da die Dialysierflüssigkeit als Substituatlösung zweifach gefiltert in das Blut überführt wird. Zum anderen ist eine Kontamination dieser Substituatlösung nicht möglich, da sie direkt in das Blut überführt und nicht mehr durch eine Substituatleitung mit darin angeordneten zusätzlichen Einrichtungen dem Blutweg zugeführt wird. Weiterhin wird durch das zumindest zeitweise erfolgende Freispülen der Membran 40 des Sterilfilters 38 und durch das ständige Freispülen der Membranen 62 und 18 des zweiten und des ersten Blutfilters 60 bzw. 12 ein Ansammeln von Keimen oder Pyrogenen an den Membranen verhindert, so daß die Gefahr einer Ruptur an einer dieser Membranen erheblich reduziert ist. Damit ist gewährleistet, daß dem Patienten stets eine völlig sterile Substituatlösung zugeführt wird. Weiterhin ist durch den Blutleckdetektor 48 eine wirkungsvolle Überwachung der Membran 18 auf Undichtigkeiten möglich.

Durch die Anordnung der Pumpe 78 in der ersten Verbindungsleitung 72 ist zum einen eine gezielte Steuerung des Substituatflusses durch die Membran 62 des zweiten Blutfilters 60 und des Filtratflusses durch die Membran 18 des ersten Blutfilters 12 durch die Vorrichtung 76 zur Steuerung der Druckdifferenz möglich. Zum anderen ist eine Umkehr des Filtrat- und Substituatflusses möglich, so daß dann das Substituat durch die Membran 18 des ersten Blutfilters 12 in das Blut überführt wird und Flüssigkeit durch die Membran 62 des zweiten Blutfilters 60 aus dem Blut herausgezogen wird. Durch eine intermittierende Schaltung des Filtrat- und Substituatflusses läßt sich zudem die Dichtheit der Membran 18 des ersten Blutfilters 12 und die Dichtheit der Membran 62 des zweiten Blutfilters 60 über den Blutleckdetektor 48 äußerst einfach und wirkungsvoll überwachen.

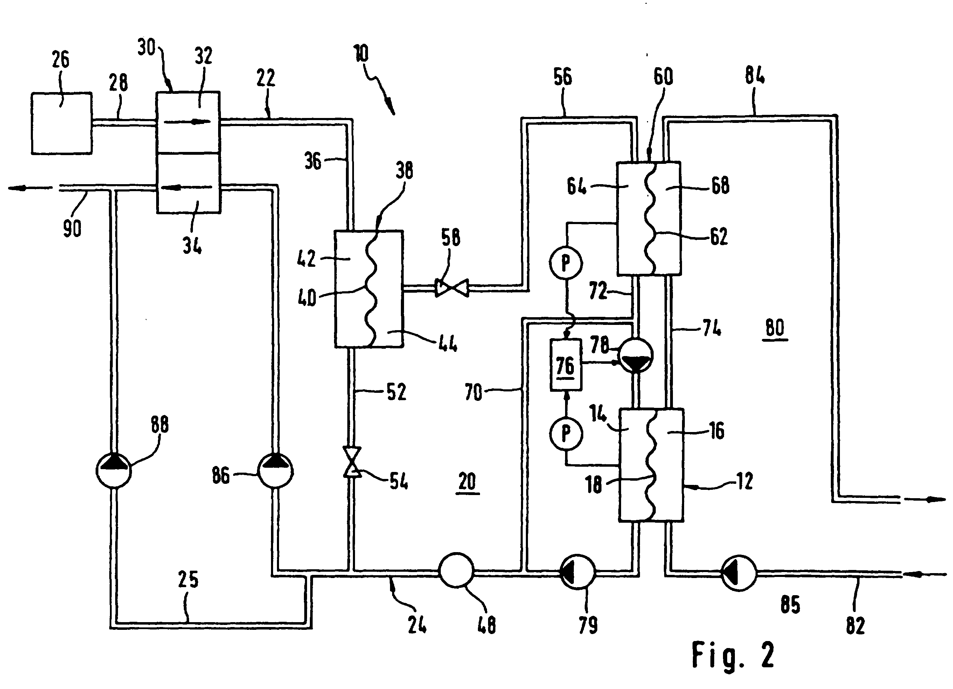

In Figur 2 ist ein zweites Ausführungsbeispiel der erfindunsgemäßen Hämo(dia)filtrationsvorrichtung 10 dargestellt, die im wesentlichen der Ausführungsform gemäß Figur 1 entspricht, so daß die gleichen Teile mit gleichen Bezugszeichen versehen sind und auf eine erneute ausführliche Beschreibung verzichtet werden kann.

Bei dieser Ausführungsform ist die Vorrichtung zur Erzeugung einer Druckdifferenz zwischen den Blutfiltern 12 und 60 dahingehend abgeändert, daß eine zweite Bypass-Leitung 70 von der ersten Verbindungsleitung 72 zwischen erster Kammer 64 des zweiten Blutfilters 60 und der Pumpe 78 zur Ableitung 24 des Dialysierflüssigkeitsweges 20 führt. In der Ableitung 24 zwischen erster Kammer 14 des ersten Blutfilters 12 und Anschluß der zweiten Bypass-Leitung 70 ist außerdem eine zweite Pumpe 79 eingeschaltet. Im Gegensatz zu der Pumpe des ersten Ausführungsbeispieles können die Pumpe 78 und die Pumpe 79 jedoch auch okkludierende Pumpen sein.

Bei diesem Ausführungsbeispiel ist ein ungestörter Fluß der Dialysierflüssigkeit durch den Dialysierflüssigkeitsweg 20 und den zweiten Blutfilter 60 möglich, wenn eine übliche Hämodialyse durchgeführt werden soll. Soll nun eine Hämodiafiltration durchgeführt werden, so wird durch Einschalten der Pumpen 78 und 79 mit jeweils unterschiedlichen Pumpraten eine Druckdifferenz zwischen dem ersten Blutfilter 12 und dem zweiten Blutfilter 60 erzeugt, so daß eine Hämodiafiltration in der schon unter Bezugnahme auf die vorhergehend beschriebenen Ausführungsbeispiele ausführlich erläuterten Weise vorgenommen werden kann.

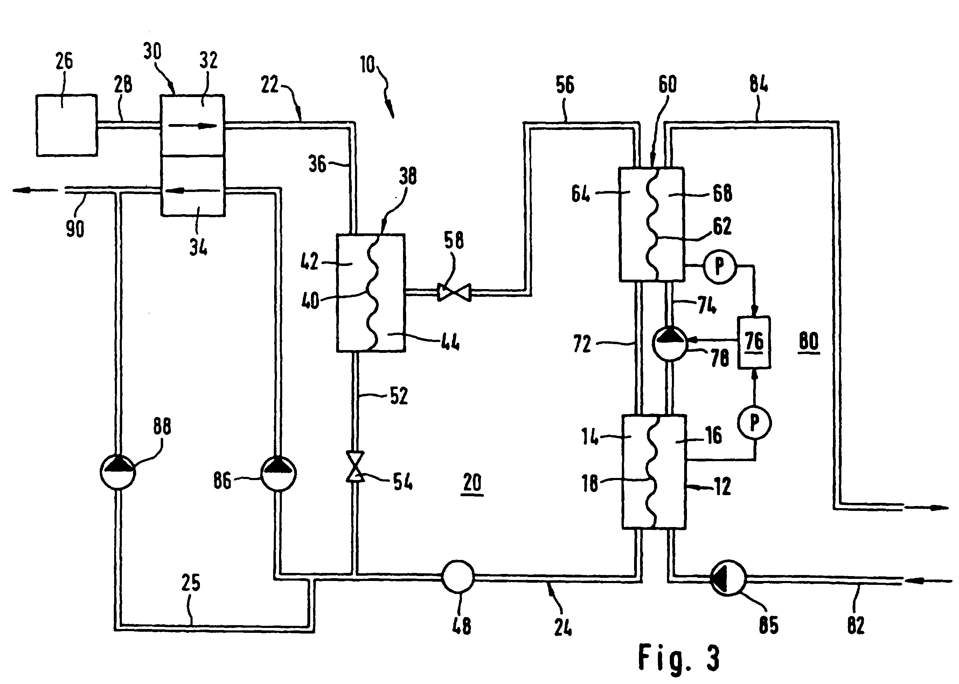

In Figur 3 ist ein drittes Ausführungsbeispiel der erfindungsgemäßen Hämo(dia)filtrationsvorrichtung 10 dargestellt. Diese entspricht ebenfalls in weiten Teilen der Ausführungsform gemäß Figur 1, so daß die gleichen Teile mit gleichen Bezugszeichen versehen sind und auf eine erneute ausführliche Beschreibung verzichtet werden kann.

Im Unterschied zu der Ausführungsform gemäß Figur 1 weist die Vorrichtung zur Erzeugung einer Druckdifferenz zwischen erstem Blutfilter 12 und zweitem Blutfilter 60 eine Pumpe 78 auf, die in die zweite Verbindungsleitung 74 zwischen zweiter Kammer 16 des ersten Blutfilters 12 und zweiter Kammer 68 des zweiten Blutfilters 60 eingeschaltet ist.

Vorteilhafterweise ist die Pumpe 78 mit einer arteriellen Blutpumpe 85 steuerungsmäßig gekoppelt, welche üblicherweise im Blutweg 80 angeordnet ist. Dabei läßt sich die Förderrate der Pumpe 78 gegenüber der Förderrate der arteriellen Blutpumpe 85 verändern. Soll eine übliche Hämodialyse durchgeführt werden, so wird die Förderrate der Pumpe 78 auf die Förderrate der arteriellen Blutpumpe 85 eingestellt. Soll dagegen eine Hämodiafiltration durchgeführt werden, so wird die Förderrate der Pumpe 78 größer oder kleiner als die der arteriellen Pumpe 85 eingestellt, je nach dem durch welchen Blutfilter der Filtrat- oder Substituatfluß geführt werden soll. Durch eine intermittierende Schaltung läßt sich zudem die Intaktheit der Membranen mittels des Blutleckdetektors 48 überprüfen.

### Bezugszeichenliste

- 10: Hämo(dia)filtrationsvorrichtung
- 12: erster Blutfilter
- 14: erste Kammer
- 16: zweite Kammer
- 18: Membran
- 20: Dialysierflüssigkeitsweg
- 22: Zuleitung
- 24: Ableitung
- 25: Ultrafiltratleitung
- 26: Dialysierflüssigkeitsquelle
- 28: erster Zuleitungsabschnitt
- 30: Bilanzierungsvorrichtung
- 32: erste Bilanziereinheit
- 34: zweite Bilanziereinheit
- 36: zweiter Zuleitungsabschnitt
- 38: Sterilfilter
- 40: Membran
- 42: erste Kammer
- 44: zweite Kammer
- 48: Blutleckdetektor
- 52: Bypassleitung
- 54: Bypassventil
- 56: dritter Zuleitungsabschnitt
- 58: Ventil
- 60: zweiter Blutfilter
- 62: Membran
- 64: erste Kammer
- 68: zweite Kammer
- 70: zweite Bypassleitung
- 72: erste Verbindungsleitung
- 74: zweite Verbindungsleitung
- 76: Vorrichtung zur Erzeugung der Druckdifferenz
- 78: erste Pumpe
- 79: zweite Pumpe
- 80: Blutweg
- 82: Blutzuleitung
- 84: Blutableitung
- 85: Blutpumpe
- 86: Dialysierflüssigkeitspumpe
- 88: Ultrafiltrationspumpe
- 90: Abfluß

## Patentansprüche

1. Hämo(dia)filtrationsvorrichtung mit einem Blutfilter, der durch eine Membran in zwei Kammern geteilt ist, wobei die erste Kammer in einen Dialysierflüssigkeitsweg und die zweite Kammer in einen Blutweg geschaltet ist, der Dialysierflüssigkeitsweg eine Zuleitung, die sich von einer Einrichtung zur Bereitstellung von Dialysierflüssigkeit bis zum Blutfilter erstreckt und in die eine erste Bilanziereinheit eingeschaltet ist, und eine Ableitung aufweist, die sich vom Blutfilter zu einem Abfluß erstreckt und in die eine zweite Bilanziereinheit eingeschaltet ist, mit einer Pumpe zum Fördern der Dialysierflüssigkeit im geschlossenen Dialysierflüssigkeitssystem, einer die Zuleitung mit der Ableitung des Dialysierflüssigkeitsweges verbindenden Bypassleitung, in die ein Bypassventil eingeschaltet ist, einer Ultrafiltrationseinrichtung, einem in der Zuleitung zwischen der ersten Bilanziereinheit und dem Blutfilter angeordneten ersten Sterilfilter, der durch eine Keime zurückhaltende Membran in eine erste Kammer und eine zweite Kammer geteilt ist, und einem zweiten Sterilfilter, der durch eine Keime zurückhaltende Membran in eine erste Kammer und eine zweite Kammer geteilt ist,
**dadurch gekennzeichnet**,
daß der zweite Sterilfilter ein weiterer Blutfilter (60) ist, welcher in der Zuleitung (22) zwischen erstem Sterilfilter (38) und erstem Blutfilter (12) angeordnet ist,
daß die erste Kammer (64) des zweiten Blutfilters (60) in den Dialysierflüssigkeitsweg (20) und die zweite Kammer (68) in den Blutweg (80) geschaltet ist,
daß die erste Kammer (64) des zweiten Blutfilters (60) über eine erste Verbindungsleitung (72) mit der ersten Kammer (14) des ersten Blutfilters (12) verbunden ist und die zweite Kammer (68) des zweiten Blutfilters (60) über eine zweite Verbindungsleitung (74) mit der zweiten Kammer (16) des ersten Blutfilters (12) verbunden ist, und
daß in einer der Verbindungsleitungen (72, 74) zwischen den Blutfiltern (60, 12) mindestens eine Pumpe (78) zur Erzeugung einer Druckdifferenz zwischen den Blutfiltern (60, 12) derart angeordnet ist, daß in dem einen Blutfilter (60, 12) ein positiver Transmembrandruck in Richtung des Blutweges (80) und in dem anderen Blutfilter (12, 60) ein negativer Transmembrandruck in Richtung des Blutweges (80) erzeugt wird.

2. Hämo(dia)filtrationsvorrichtung nach Anspruch 1, **dadurch gekennzeichnet**, daß der erste Sterilfilter (38) in die Zuleitung (22) eingeschaltet ist und die erste Kammer (42) des ersten Sterilfilters (38) zumindest teilweise in Durchfluß schaltbar ist.

3. Hämo(dia)filtrationsvorrichtung nach Anspruch 2, **dadurch gekennzeichnet**, daß die erste Kammer (42) des ersten Sterilfilters (38) mit der zur Ableitung (24) führenden Bypass-Leitung (52) verbunden ist.

4. Hämo(dia)filtrationsvorrichtung nach Anspruch 3, **dadurch gekennzeichnet**, daß in dem Zuleitungsabschnitt (56) stromab des ersten Sterilfilters (38) ein Absperrorgan (58) eingeschaltet ist.

5. Hämo(dia)filtrationsvorrichtung nach Anspruch 1, **dadurch gekennzeichnet**, daß die Pumpe (78) in die erste Verbindungsleitung (72) zwischen der ersten Kammer (14) des ersten Blutfilters (12) und der ersten Kammer (64) des zweiten Blutfilters (60) eingeschaltet ist, und daß eine zweite Bypass-Leitung (70) von der ersten Verbindungsleitung (72) zwischen zweitem Blutfilter (60) und Pumpe (78) zur Ableitung (24) des Dialysierflüssigkeitsweges (20) führt.

6. Hämo(dia)filtrationsvorrichtung nach Anspruch 5, **dadurch gekennzeichnet**, daß eine zweite Pumpe (79) in die Ableitung (24) zwischen erstem Blutfilter (12) und Anschluß der zweiten Bypass-Leitung (70) eingeschaltet ist.

7. Hämo(dia)filtrationsvorrichtung nach Anspruch 1, **dadurch gekennzeichnet**, daß die Pumpe (78) in die zweite Verbindungsleitung (74) zwischen der zweiten Kammer (16) des ersten Blutfilters (12) und der zweiten Kammer (68) des zweiten Blutfilters (60) eingeschaltet ist.

8. Hämo(dia)filtrationsvorrichtung nach einem der vorhergehenden Ansprüche, **dadurch gekennzeichnet**, daß die Pumprate und die Pumprichtung der Pumpe (78) so einstellbar ist, daß in Blutströmungsrichtung im ersten Filter (12) ein positiver Transmembrandruck der Blut- und Dialysierflüssigkeitsseite und im nachgeschalteten zweiten Filter (60) ein negativer Transmembrandruck zwischen der Blut- und Dialysierflüssigkeitseite erzeugt wird.

9. Hämo(dia)filtrationsvorrichtung nach einem der vorangehenden Ansprüche, **gekennzeichnet durch** Mittel zur Überwachung der Druckdifferenz zwischen erstem Blutfilter (12) und zweitem Blutfilter (60).

10. Hämo(dia)filtrationsvorrichtung nach einem der vorangehenden Ansprüche, **gekennzeichnet durch** Mittel (76) zur Steuerung der Druckdifferenz zwischen erstem Blutfilter (12) und zweitem Blutfilter (60).

## Claims

1. Haemo(dia)filtration device with a blood filter, divided by a membrane into two chambers, whereby the first filter is connected to a dialysis fluid line and the second is connected to a blood line, the dialysis fluid line has a feed line which extends from a device for preparation of the dialysis fluid to the blood filter and is connected to the a first balance, and a drain line, which extends from the blood filter to an outlet and is connected to a second balance, with a pump to convey the dialysis fluid in a sealed dialysis fluid system, a bypass line joining the feed line to the drain line of the dialysis fluid line, an ultra-filtration device, a first sterile filter arrange don the feed line between the first balance and the blood filter, divided into a first chamber and a second chamber by a membrane which retains germs, and a second sterile filter, divided by a germ-retaining membrane into a first chamber and a second chamber.
**characterised by the fact that**
the second sterile filter is another blood filter (60), which is arranged in the feed line (22) between the first sterile filter (38) and the first blood filter (12);
the first chamber (64) of the second blood filter (60) is connected in the dialysis fluid line (20) and the second chamber (68) is connected in the blood line (80);
the first chamber (64) of the second blood filter (60) is connected by a first connecting line (72) with the first chamber (14) of the first blood filter (12) and the second chamber (68) of the second blood filter (60) is connected via a second connecting line (74) with the second chamber (16) of the first blood filter (12) and
in one of the connecting lines (72, 74) between the blood filters (60, 12) at least one pump (78) is arranged to produce a differential pressure between the blood filters (60, 12) such that in one of the blood filters (60, 12) a positive trans-membrane pressure is produced in the direction of the blood line (8) and in the other blood filter (12, 60) a negative trans-membrane pressure is produced in the direction of the blood line (80).

2. Haemo(dia)filtration device as per claim 1, **characterised by the fact** that the first sterile filter (38) is connected in the feed line (22) and the first chamber (42) of the first sterile filter (38) is connectable at least partially as a through-flow.

3. Haemo(dia)filtration device as per claim 2, **characterised by the fact** that the first chamber (42) of the first sterile filter (38) is connected to the bypass line (52) leading to the drain line (24).

4. Haemo(dia)filtration device as per claim 3, **characterised by the fact** that in the feed line section (56) a barrier element (58) is connected downstream of the first sterile filter (38).

5. Haemo(dia)filtration device as per claim 1, **characterised by the fact** that the pump (78) is connected in the first connecting line (72) between the first chamber (14) of the first blood filter (12) and the second chamber (64) of the second blood filter (60) and that a second bypass line (70) leads from the first connecting line (72) between the second blood filter (60) and pump (78) to the drain line of the dialysis fluid line (20).

6. Haemo(dia)filtration device as per claim 5, **characterised by the fact** that a second pump (79) is connected in the drain line (24) between the first blood filter (12) and the connection to the second bypass line (70).

7. Haemo(dia)filtration device as per claim 1, **characterised by the fact** that the pump (78) is connected in the second connecting line (74) between the second chamber (16) of the first blood filter (12) and the second chamber (68) of the second blood filter (60).

8. Haemo(dia)filtration device as per one of the above claims, **characterised by the fact that** the pump rate and pump direction of the pump (78) are adjusted such that in the blood flow direction in the first filter (12) there is a positive trans-membrane pressure on the blood and dialysis fluid side and in the second filter connected downstream (60), a negative trans-membrane pressure is produced between the blood and dialysis fluid side.

9. Haemo(dia)filtration device as per one of the above claims, **characterised by** a device for monitoring the pressure differential between the first blood filter (12) and the second blood filter (60).

10. Haemo(dia)filtration device as per one of the above claims, **characterised** by a device (76) for controlling the pressure differential between the first blood filter (12) and the second blood filter (60).

## Revendications

1. Dispositif d'hémo(dia)filtration avec un filtre à sang, qui est partagé par une membrane en deux chambres, dans lequel la première chambre est montée dans un circuit de liquide de dialyse et la deuxième chambre dans un circuit de sang, le circuit de liquide de dialyse est relié à une canalisation qui vient d'un dispositif de préparation de liquide de dialyse jusqu'au filtre à sang et est relié à une première unité d'équilibrage, et présente une canalisation d'évacuation qui s'étend du filtre à sang jusqu'à une vidange et est connectée à une deuxième unité d'équilibrage, avec une pompe pour alimenter le liquide de dialyse dans le système fermé de liquide de dialyse, une canalisation de by-pass reliant la canalisation d'entrée à la canalisation d'évacuation du circuit de liquide de dialyse, dans laquelle est intercalée une vanne de by-pass, avec un dispositif d'ultrafiltration, un premier filtre stérile disposé dans la canalisation entre la première unité d'équilibrage et le filtre à sang, qui sépare une première chambre et une deuxième chambre au moyen d'une membrane retenant les germes, et un deuxième filtre stérile qui sépare une première chambre et une deuxième chambre au moyen d'une membrane retenant les germes,
caractérisé en ce que,
le deuxième filtre stérile est une autre filtre à sang (60), qui est disposé dans la conduite d'alimentation (22) entre le premier filtre stérile (38) et le premier filtre à sang (12),
la première chambre (64) du deuxième filtre à sang (60) est connectée au deuxième filtre à sang (60) dans le circuit de liquide de dialyse (20) et la deuxième chambre (68) dans le circuit de sang (80) ,
la première chambre (64) du deuxième filtre à sang (60) est reliée à travers une première canalisation de liaison (72) avec la première chambre (14) du premier filtre à sang (12) et la deuxième chambre (68) du deuxième filtre à sang (60) est reliée à travers une deuxième canalisation de liaison (74) avec la deuxième chambre (16) du premier filtre à sang (12), et
dans une des canalisations de liaison (72, 74) entre les filtres à sang (60, 12) est disposée au moins une pompe pour produire une différence de pression entre les filtres à sang (60, 12) de manière à ce que soit produit dans un filtre à sang (60, 12) une pression transmembranaire positive dans la direction du circuit de sang (80) et dans l'autre filtre à sang (12, 60) une pression transmembranaire négative dans la direction du circuit de sang (80).

2. Dispositif d'hémo(dia)filtration selon la revendication 1, caractérisé en ce que, le premier filtre stérile (38) est monté dans la conduite d'alimentation (22) et la première chambre (42) du premier filtre stérile (38) est commutable au moins partiellement en circulation.

3. Dispositif d'hémo(dia)filtration selon la revendication 2, caractérisé en ce que, la première chambre (42) du premier filtre stérile (38) est reliée avec la canalisation de by-pass (52) conduisant à la canalisation d'évacuation (24).

4. Dispositif d'hémo(dia)filtration selon la revendication 3, caractérisé en ce que, un organe d'arrêt (58) est connecté dans le tronçon d'alimentation (56) en aval du premier filtre stérile (38).

5. Dispositif d'hémo(dia)filtration selon la revendication 1, caractérisé en ce que, la pompe (78) est connectée dans la canalisation de liaison (72) entre la première chambre (14) du premier filtre à sang (12) et la première chambre (64) du deuxième filtre à sang (60), et en ce qu'une deuxième canalisation de by-pass (70) conduit de la première canalisation de liaison (72) entre le deuxième filtre à sang (60) et la pompe (78) à la canalisation d'évacuation (24) du circuit de liquide de dialyse (20).

6. Dispositif d'hémo(dia)filtration selon la revendication 5, caractérisé en ce qu'une deuxième pompe (79) est connectée entre le premier filtre à sang (12) et la connexion de la deuxième canalisation de by-pass (70).

7. Dispositif d'hémo(dia)filtration selon la revendication 1, caractérisé en ce que, la pompe (78) est connectée dans la deuxième canalisation de liaison (74) entre la deuxième chambre (16) du premier filtre à sang (12) et la deuxième chambre (68) du deuxième filtre à sang (60).

8. Dispositif d'hémo(dia)filtration selon l'une des revendications précédentes, caractérisé en ce que, la vitesse de pompage et la direction de pompage de la pompe (78) est ajustable de sorte qu'on produise dans la direction de circulation du sang dans le premier filtre (12) une pression transmembranaire positive entre le côté du liquide de dialyse et le sang et dans le deuxième filtre (60) connecté à la suite une pression transmembranaire négative entre le côté du liquide de dialyse et le sang.

9. Dispositif d'hémo(dia)filtration selon l'une des revendications précédentes, caractérisé par des moyens de surveillance de la différence de pression entre le premier filtre à sang (12) et le deuxième filtre à sang (60).

10. Dispositif d'hémo(dia)filtration selon l'une des revendications précédentes, caractérisé par des moyens (76) de régulation de la différence de pression entre le premier filtre à sang (12) et le deuxième filtre à sang (60).
